# EUROPEAN PATENT SPECIFICATION

(11) **EP 3 133 157 B1**
(45) Date of publication and mention of the grant of the patent: **08.05.2019**
(21) Application number: 16190737.3
(22) Date of filing: 30.03.2011
(51) Int. Cl.: C12N 9/64

(54) **A PROCESS FOR PURIFYING VITAMIN K DEPENDENT PROTEINS**
VERFAHREN ZUR REINIGUNG VON VITAMIN-K-ABHÄNGIGEN PROTEINEN
PROCÉDÉ PERMETTANT DE PURIFIER DES PROTÉINES DÉPENDANTES DE LA VITAMINE K

(30) Priority: 30.03.2010 EP 10158511; 16.04.2010 US 282895 P
(43) Date of publication of application: 22.02.2017
(62) Divisional of application: 11713216.7
(73) Proprietor: Octapharma AG, 8853 Lachen (CH)
(72) Inventor: GILLJAM, Gustav, 14242 Stockholm (SE); WINGE, Stefan, 12050 Årsta (SE)
(74) Representative: Patent- und Rechtsanwälte Ullrich & Naumann

(56) References cited:
- EP-A1- 2 027 875
- WO-A1-2009/156430
- ARAKAWA T ET AL: "MEP HyperCel chromatography II: Binding, washing and elution", PROTEIN EXPRESSION AND PURIFICATION, ACADEMIC PRESS, SAN DIEGO, CA, vol. 71, no. 2, 12 November 2009 (2009-11-12), pages 168-173, XP026969372, ISSN: 1046-5928, DOI: DOI:10.1016/J.PEP.2009.11.004 [retrieved on 2009-11-12]
- KALEAS K A ET AL: "Industrial case study: Evaluation of a mixed-mode resin for selective capture of a human growth factor recombinantly expressed in E. coli", JOURNAL OF CHROMATOGRAPHY, ELSEVIER SCIENCE PUBLISHERS B.V, NL, vol. 1217, no. 2, 8 January 2010 (2010-01-08), pages 235-242, XP026817106, ISSN: 0021-9673 [retrieved on 2009-07-17]
- TSUMOTO KOHEI ET AL: "Review: Why is Argine Effective in SUppressing Aggregation?", PROTEIN AND PEPTIDE LETTERS, BENTHAM SCIENCE PUBLISHERS, NL, vol. 12, no. 7, 1 October 2005 (2005-10-01), pages 613-619, XP009090675, ISSN: 0929-8665, DOI: DOI:10.2174/0929866054696109

## Description

The present invention pertains to a process of purifying vitamin K dependent proteins such as coagulation factor VII by the process of the invention.

Hemophilia is a group of hereditary genetic disorders that impair the body's ability to control blood clotting or coagulation. In one of its forms, Hemophilia B, clotting factor IX (FIX) is deficient, Hemophilia B occurs in about 1 in 25,000 male births. Factor IX (or Christmas factor) is one of the serine proteases of the coagulation system. It is a vitamin K-dependent plasma protein that participates in the intrinsic pathway of blood coagulation by converting factor X to its active form in the presence of Ca²⁺ ions, phospholipids, and factor VIIIa. The FIX protein is an essential factor in blood coagulation with multifunctional properties.

Factor IX (FIX) is a single-chain glycoprotein containing 461 amino acids. It is synthesized primarily in the liver and secreted in plasma. The factor IX molecule consists of several discrete functional domains, including a signal peptide, propeptide, a Gla domain, two epidermal growth factor-like (EGF) domains, an activation peptide and a catalytic, trypsin-like domain (serine protease domain) (The propeptide is cleaved prior to secretion to generate the mature 415 aa FIX molecule. The protein is further processed into an active form, to a heterodimer consisting of a light chain and a heavy chain linked by a disulfide bond.

The deficiency of FIX can be treated with plasma-derived concentrates of FIX or with recombinantly produced FIX. The treatment with FIX concentrates has led to a normalized life of the hemophilia patients. Historically, Hemophilia B has been treated with FIX originating from human blood plasma. In blood plasma, under normal conditions, the FIX molecule is circulating in its native form, whereas it is activated through a complicated process initiated in the blood cascade of coagulation enzymes.

Plasma derived FIX products occur on the market with different purities, depending on which purification method is applied. The methods used to purify FIX were normally a combination of different chromatography steps (mainly ion exchange and affinity steps, for purifying and ultra-filtration step (s) for concentration / desalting of the product.

Harrison *et al.* (S. Harrison et al. Sem Hematol 35 (Suppl 2): 4-10 (1998) describes the manufacturing process for a recombinant FIX (Benefix®) produced in CHO cells. The cells are harvested by microfiltration and subsequently concentrated in an ultrafiltration/diafiltration step where the buffer is exchanged to a Tris buffer to provide a consistent buffer for loading onto the first chromatography column. Four independent chromatography steps is used for the purification of rFIX, the first is an anion exchange step (Q Sepharose FF), which is performed in a pseudoaffinity mode. The column is eluted with 10 mM calcium chloride at pH 8.0. The calcium chloride induces a conformational change in the FIX molecule, which causes it to detach from the column.

The Q Sepharose step is followed by purification on a Matrex cellufine sulfate column, which is a heparin analogue used for affinity purification of proteins with heparin-binding domains. It also behaves as a cation exchange resin due to the negatively charged sulfate groups. The cellufine purification step removes low levels of HCPs.

The cellufine eluate is loaded onto a Ceramic-Hydroxyapatite column, which is a synthetic form of calcium phosphate. It is used to separate proteins of varying charges and it provides the possibility to remove lower specific activity forms of rFIX. This column is eluted with a stepwise elution by increasing the phosphate concentration to a final concentration of 0.5 M.

The final purification step in the manufacturing process of Benefix® is Chelate-EMD-Cu(II). Proteins interacts with the immobilized metals retained by the resin. Bound rFIX is eluted with imidazole as a displacer and trace contaminants is removed in this purification step, which is followed by a virus filtration step (Viresolve-70) and finally by a ultrafiltration/diafiltration step where rFIX is concentrated and the buffer is exchanged to the formulation buffer.

The above-described rFIX manufacturing process is consistent, 65 batches have been analysed and the specific activity was found to be 276 ± 23 IU/mg. The Gla content was 11.4±0.1 mol Gla/mol rFIX and the total impurities were found to be 0.01±0.01 % and 0.03±0.01 % determined by RP-HPLC and HCP-ELISA, respectively.

Lindsay et al. (J Chrom A 1026: 149-157 (2004)) describes a method to purify rFIX from transgenic pig milk. Heparin Sepharose FF was used as the first step in the purification scheme, followed by an anion exchange step.

The heparin-binding domain of factor IX is located in the C-terminal end of the molecule. This region lacks PTMs and thus the heparin chromatography step allows the entire population of rFIX to be isolated. The specific activity of the eluate was 30-35 IU/mg, which indicates that a large fraction is inactive. Active rFIX subpopulations were separated from inactive subpopulations during a gradient elution of the AIEX column.

Kaufman et al. (JBC 261:9622-9628 (1986)) describes expression of rFIX in CHO cells and purification of the protein. The rFIX containing cell culture medium was concentrated by ultrafiltration and dialyzed against a buffer containing 3 mM CaCl₂, 0.05 M Tris-HCI and 0.5 M NaCl prior to application to an immunoaffinity column with conformation specific antibodies (anti-FIX:Ca(II) antibodies). The column was subsequently eluted with 10 mM EDTA, 0.05 M Tris-HCI and 0.15 M NaCl.

The presence of 3 mM CaCl₂ in the starting material prior to application to the column resulted in a separation between active and inactive species of FIX. The inactive species failed to bind to the column and active FIX could be eluted from the column with EDTA.

WO-A-2009/007451 discloses a purification method of FVIII using a mixed-mode or multimodal resin. The purification method is based on contacting FVIII protein with a multimodal or mixed-mode resin containing ligands which comprise a hydrophobic part and a negatively charged part and eluting said FVIII protein with an elution buffer containing at least 1.5 M salt and at least 40% (w/v) of ethylene glycol, propylene glycol or a mixture thereof, and calcium ions.

EP-A-1 707 634 discloses a method for isolation of recombinantly produced proteins i.e. by various methods such as immuno-affinity chromatography, affinity chromatography, protein precipitation, buffer exchanges, ionic exchange chromatography, hydrophobic interaction chromatography, mixed-mode hydrophobic/ion exchange chromatography media, chelating chromatography, carbohydrate affinity like lectin or heparin affinity chromatography, size-exclusion chromatography, electrophoresis, dialysis, different precipitation agents such as polyethylene glycol, ammonium sulphate, ethanol, hydroxy apatite adsorption, filter membrane adsorption, ligands coupled to magnetic particles etc. However, it is identifying no particular chromatographic purification steps.

WO-A-2005-082483 discloses a process for the purification of antibodies from one or more impurities in a liquid, which process comprises contacting said liquid with a first chromatography resin comprised of a support to which multi-modal ligands have been immobilised to adsorb the antibodies to the resin, wherein each multi-modal ligand comprises at least one cation-exchanging group and at least one aromatic or hetero aromatic ring system. An eluent is added to release the antibodies from the resin and the eluate is contacted with a second chromatography resin.

WO-A-2005/121163 discloses a process for the isolation of one or more proteins from a protein solution. The process comprises the steps of providing a protein solution comprising one or more specific proteins and having a pre-set pH and a pre-set ionic strength or conductivity, applying the protein solution to an adsorption column comprising a particle with at least on high density non-porous core surrounded by a porous material, the adsorbent comprises a particle density of at least 1.5 g/ml and a mean volume particle diameter of at most 150um. The column is optionally washed before eluting the protein (s) from the adsorbent.

WO-A-2009/156430A1 discloses a purification method of FVIII using a mixed-mode or multimodal resin. The purification method is based on contacting FVIII protein in a solution having a high ionic strength with a multimodal or mixed-mode resin containing ligands, which comprise a hydrophobic part and a negatively charged part, and eluting said FVIII protein with an elution buffer comprising at least one amino acid, which is positively charged at pH 6-8.

EP 2 027 875 A1 discloses a process for isolation and purification of a target protein free pf prion protein (PrPSC) using a mixed-mode or multimodal resin. The purification method is based on contacting a potentially PrPSC-contaminated sample comprising a target protein with a multimodal chromatographic material, setting the buffer condition so that the target protein is bound to the multimodal chromatographic material and PrPSC are eluted, whereby, following the PrPSC elution the target protein may be eluted under certain elution buffer conditions.

Arakawa et al. 2009 (Protein Expression And Purification, Academic Press, San Diego, vol. 71, no 2, pages 168-173) is the second of a two-article series considering the properties of the specific MEP HyperCel chromatography matrix in the context of monoclonal antibody purification. In particular, the article considers the specific binding, washing and elution conditions suitable for monoclonal antibody purification.

Kaleas et al. 2010 (Journal Of Chromatography, Elsevier Science Publishers B.V., vol. 1217, no. 2, pages 235.242) provides an industrial case study in which a mixed-mould resin is evaluated for selective capture of recombinant human vascular endothelial growth factor (rhVEGF) expressed in *E. coli* bacteria. Benefits of the mixed-mode resin, when used to purify the rhVEGF protein expressed as inclusion bodies, harvested from the cell lysate with the solids and subsequently solubilised and refolded at a pH of 9.8 in the presence of urea and redox agents, are described.

Tsumoto et al. 2005 (Protein And Peptide Letters, Bentham Science Publishers, vol 12., no. 7, pages 613- 619) is arewview article considering the effects of arginine in supressing aggregation of folding intermediates during protein refolding. Further, the review considers the effects of arginine on the stability and solubility of pure proteins but concludes that there is no explanantion as to why arginine suppresses aggregation of folding intermediates.

In order to improve FIX containing products, specific affinity chromatography directed against the FIX molecule was employed, which effectively removed contaminants to a high degree of FIX purity. The disadvantage with the often-used immuno affinity chromatography was that it is relatively expensive and that the monoclonal antibodies used as affinity ligands, where of animal origin.

In the mid-1980s, there were some virus transmissions associated with plasma derived FIX products. Even if this problem was solved through implementation of specific virus reduction steps, this was the starting point of the development of recombinant FIX products (rFIX). In the 1990s, the first rFIX product was marketed and up to date the only one.

One object of the invention was to provide a process by which the drawbacks of the purification processes of vitamin K dependent proteins in prior art could be avoided. It is known from prior art that one disadvantage of traditional ion exchange chromatography resins (as for example SP-, CM-, Q- or DEAE Sepharose FF ion exchange chromatography resins) is that the binding of protein to the resin only can be performed within relatively low salt concentration (conductivity) and pH, typically in the range of 0.01-0.15M of salt (NaCl etc.) concentration. The optimal pH range is dependent if a cation exchanger or an anion exchanger is used and the iso-electric point of the target product to be bound on the resin. In general the pH range in which a target protein binds to an ion exchange resin is typically approximately one pH unit up or down from the iso-electrical point of the target product.

In certain applications there would be a demand to be able to use the relatively mild purification conditions an ion exchange chromatography step exerts towards the proteins, also directly (without further dilution) to a chromatography resin at somewhat increased ionic strength and at a pH out of the normal range which cannot be used for conventional ion exchange chromatography.

Multimodal (or mixed mode) chromatography is a tool for purifying proteins. Described in, for example, Manufacturer data sheet GE Healthcare (11-0035-45AA) Capto Adhere, Manufacturer data sheet GE Healthcare (28-9078-88AA) Capto MMC and WO-A-2009/024620 "A process for the isolation and purification of a target protein, free of prion proteins". A disadvantage is that the elution often includes relatively harsh conditions like for example pH below or above neutral pH, alone or in combination with other elution parameters such as for example ethylene glycol, and as for example described in WO-A-2009/007451.

An increased ionic strength can be of significant advantage for the protein stability in a protein solution, especially in a crude protein preparation like in the harvest of recombinant proteins or in plasma derived products where potential proteases are present in the solution, which can affect the target protein negatively.

As proteases often work best at physiological conditions (like is the case in most cell systems), i.e. approximately pH 7 and a salt concentration of approximately 0.15M, it can be advantageous to change these conditions to minimize the effects of proteases. Such changes can typically be performed by addition of salt and/or change of the pH. Both of these parameters are critical for the performance of a conventional ion-exchange chromatography step and thus often impossible to achieve. Thus another object of the invention was to provide a process in which this problem has been solved and rendering possible to add salt and/or change the pH in crude protein samples comprising potentially proteolytic factors such as proteases which could degrade the target protein. The process should further be able to process the protein solution with as less as possible further measures and to bind the target protein to a chromatography resin. This would provide an optimized step of concentration and purification of the target protein from a crude sample or further purification downstream using e.g. other chromatography steps such as affinity chromatography.

This need is of specific importance, because degradation of the target protein during purification would be prevented or at least diminished.

This makes it possible to remove proteases and other contaminants, which might be present in a crude harvest, before elution of the target protein.

Another object of the invention was to provide a process of purifying factor VII, in particular starting from a cell culture harvest of recombinant factor VII.

Seven plasma glycoproteins are known to be dependent on vitamin K for their biosynthesis. They are prothrombin (factor II), factor VII, factor IX, factor X, protein C, protein S and protein Z. The Gla domain is a common structural feature in all these vitamin K-dependent proteins and immediately after the Gla domain, each of the proteins (except prothrombin) has one or more EGF-like domains. The vitamin K-dependent proteins require Ca2+ ions to express their physiological function and the calcium binding sites involve at least the Gla domain and the EGF-like domains. Calcium binding enables these proteins to bind to phospholipids/cell membranes and thus express their full biological activities. According to the invention the above-stated object is accomplished by a process of manufacturing a prion-free Vitamin K dependent protein in a purification sequence employing chromatography characterized in that
- at least one chromatography step is performed using a multimodal resin;
- providing a fraction containing Vitamin K dependent protein in an aqueous solution;
- contacting the fraction containing the Vitamin K dependent protein with a multimodal resin at a pH between 6-9;
- optionally washing the multimodal resin having the Vitamin K dependent protein adsorbed with an aqueous washing buffer to wash away contaminants and retain the Vitamin K dependent protein, before the Vitamin K dependent protein is eluted;
- the Vitamin K dependent protein is eluting from the multimodal resin at a pH between 6 to 9 in a buffer comprising arginine; and
- optionally collecting Vitamin K dependent protein containing fractions in purified or enriched form,
wherein the Vitamin K dependent protein is factor VII.

The present invention using a multimodal resin as a capture purification step provides the possibility to adjust the salt concentration and pH to values, which minimize the risk of active proteases in the protein solution during binding of the target protein to the multimodal chromatography step.

An advantage of the process of the invention is the possibility to further apply a wash step before elution of the target protein after binding of the target protein to the multimodal resin for example used as a capture step in a recombinant process. This is achieved by using the multimodal resin was to wash the resin by selecting a suitable washing buffer to remove proteases and other contaminants which adheres to the multimodal resin, before eluting the target protein. The suitable wash buffer preferably is containing a salt or an amino acid or a buffer component, or mixtures thereof at a pH suitable to still inhibit protease activity during the wash removal step.

The process of the invention renders possible to elute the target protein, bound to the multimodal resin, in a mild elution environment with distinct elution properties (i.e in a volume as small as possible). This was achieved by using an increased pH or an increased salt concentration or addition of an amino acid or combination thereof. The process of the invention provides advantages such as inhibition of aggregates, preserving the native molecule structure and providing a low volume for the further downstream processing.

The present invention also facilitates a process of purification without addition of human or animal derived stabilizing additives and the use of a whole process which is absent thereof (monoclonal antibody based immuno affinity resins). The use of the multimodal resin, in particular as capture step, facilitates also a higher binding capacity in comparison with conventional ion exchangers, which results in a more concentrated product eluate from the column, which can be of advantage for the product stability.

According to one embodiment of the invention, the multimodal chromatography may be performed in a chromatographic column. This may be regarded as a first capture step. The process of the invention can also be performed in a batch mode.

In one embodiment of the invention the chromatography on multimodal resins is combined with a chromatography on a resin having a yeast derived affinity ligand, employing a purity of >90% after elution of the factor VII.

In another further embodiment of the invention the multimodal resin step and the yeast derived affinity ligand chromatography step is combined with other chromatography purification step to exert a purity of more than 99% in the final factor VII product.

In another embodiment of the invention, the multimodal resin comprises moieties bound to a matrix and the moieties are able to interact with factor VII in a mixture by ionic interactions and other types of interactions such as hydrogen bonding, hydrophobic and thiophilic interactions.

In a further embodiment of the invention, the affinity ligand is a yeast derived F_{ab} fragment directed towards factor VII.

In a further embodiment of the invention, the multimodal resin step is performed to capture the factor VII from a crude protein solution where after processing the resulting multimodal chromatography resin eluate on the yeast derived affinity ligand chromatography step and after elution of factor VII from said affinity chromatography step, exerting a purity of more than 90% in relation to proteins and DNA.

In another further embodiment, the invention is characterised in that the purity of the final product is more than 99%, if the multimodal resin step performed to capture the factor VII from a crude protein solution, where after processing the resulting multimodal chromatography resin eluate is further processed on additional chromatography step(s) selected from size exclusion, anion exchange, cation exchange, hydrophobic interaction and immobilized metal affinity chromatography and a yeast derived affinity ligand.

In still another embodiment of the invention, the factor VII is present in a solution.

In yet another embodiment of the invention, the factor VII is in a crude protein solution including potentially proteases, which can degrade the product.

It can be advantageous to apply the washing buffer to the multimodal resin, to wash away contaminants (proteases, DNA etc.) and retain the factor VII, before it is released.

In an additional embodiment of the invention, the multimodal resin is washed with a wash buffer at a pH between 6-9, before eluting the factor VII.

In a further embodiment of the invention, the elution is performed with arginine as the elution agent. According to the invention, the elution agent can be combined with an increased salt concentration in which the salt is selected from the Hofmeister series. According to the invention, the elution can take part either with arginine alone or in combination with increased salt concentration or only with increased salt concentration, all of this within the pH range between 6-9, preferably at pH 7.0.

According to the invention NaCl and KCI are preferred in regard of salt comprised in the Hofmeister series, which for example are sodium, potassium, ammonium, magnesium, calcium, barium, acetate, phosphate and sulphate.

According to the invention, the concentration of arginine is in particular in the range of from about 0.1M to about 2M and 0.1 M to 4M for the salt according to Hofmeister series; in particular in the range of from about 0.4M to about 1.5M for arginine and 0.6 M to 2M for the salt according to Hofmeister series or in the range of from about 0.3M to about 0.7M for arginine and 0.8 M to 1.2 M for the salt according to Hofmeister series

According to another embodiment of the invention, the factor VII binds to the multimodal resin at a pH between 6-9, preferably at pH 7.0.

In a further embodiment of the invention, a buffering substance is used comprising preferably at least one of the substances selected from the group consisting of sodium citrate, histidine, 2-(4-(2-Hydroxyethyl)-1-piperazinyl)-ethane sulfonic acid (HEPES), 2-(N-Morpholino)ethane sulfonic acid (MES), Tris base and sodium acetate in particular in a range of about pH 6 to about pH 9.

In the process of the invention, a non-ionic detergent can be present in any of the buffers used, which non-ionic detergent is in particular selected from the group consisting of Polysorbates (Polysorbate 20, 40, 60, 80) and Pluronic F68.

In the elution buffer with a pH 6-9, preferably at pH 7.0, the amount of arginine is typically in the range of 0.1 to 2M, in particular 0.5M.

In the elution buffer with a pH 6-9, preferably pH 7.0, sodium chloride is included in a range of 0.1-4M, in particular in a range from 0.05 to 0.3M.

In the elution buffer with a pH 6-9, preferably at pH 7.0, arginine and sodium chloride is included in a range of 0.1-0.5M, in particular in a range from 0.3 to 0.7M.

In the wash buffer with a pH 6-9, preferably pH 7.0, sodium chloride is included in a range of 0.01-0.3M, in particular in a range from 0.05 to 0.15M.

The amount of non-ionic detergent is typically in the range of 0.001 to 1%, in particular in the buffers for multimodal chromatography 0.02%.

The multimodal chromatography resin, which can be employed according to the invention, may contain at least one of the following moieties:
a. a positively charged N-Benzyl-N-methyl ethanolamine ligand,
b. a negatively charged 2- (benzoylamino) butanoic acid ligand,
c. a phenylpropyl ligand,
d. a N-hexyl ligand,
e. a 4-Mercapto-Ethyl-Pyridine ligand,
f. a 3-((3-methyl-5-((tetrahydrofuran-2-ylmethyl)-amino)-phenyl)-amino)-benzoic acid ligand or combinations thereof.

In particular, a multimodal chromatography resin for use according to the present invention is selected from the following commercially available resins HEP Hypercel™; PPA Hypercel™; Capto Adhere™; Capto MMC™; MEP Hypercel™.

In another embodiment of the process of the invention, the multimodal chromatography step is combined with a factor VII affinity chromatographic step wherein the affinity is provided by a protein ligand such as an antibody fragment, which is expressed e.g. in yeast.

In another embodiment of the present invention, the purification sequence may further comprise pathogen removal/inactivation steps comprising a chemically based inactivation step, a size based removal step, chromatography steps or combinations thereof, which steps are based on different physiological properties directed to the pathogen to be removed. An example of a pathogen inactivation step well described in literature is the chemically based solvent detergent method, for example based on tri-n-butyl phosphate and Triton X-100, which disrupt all lipid-enveloped viruses, disclosed in EP-A-131 740. An example of a pathogen removal step based on size, is for example a nanofilter with a mean pore size of app. 20nm, such as a Planova 20 filter. Another example of pathogen removal is based on chromatography. For example, affinity chromatography is known to exert pathogen removal properties in general, for example a yeast derived factor VII affinity ligand chromatography resin.

In a particular embodiment of the process of the invention, the purification sequence further comprises the following steps:
1. a cation multimodal resin such as Capto MMC;
2. a chemically based inactivation step for lipid enveloped viruses in particular the solvent/detergent-inactivation employing tri-n-butyl phosphate and Triton X-100 as disclosed in EP-A-131 740;
3. an affinity resin based on a ligand expressed in yeast;
4. a cation exchanger such as SP Sepharose or Resource S;
5. a pathogen filtration removal step with a mean pore sized of about 20nm such as Planova 20N;
6. a buffer exchange and/or concentrating step such as ultra-filtration with an approximate cut off of 10kDa;
7. a size exclusion chromatography resin such as Superdex 200.

In a particular embodiment, the process of the invention could comprise the following steps:
- A Capto MMC™ multi modal resin was packed in a column and equilibrated with a buffer comprising 20mM sodium citrate, 0.1M NaCl, 0.02% Polysorbate 80 at pH 7.0.
- The crude factor VII containing sample was applied comprising approximately the same conditions as in the equilibration buffer above and the target protein and some additional impurity proteins did bind to the column.
- A washing step, according to the invention, using the equilibration buffer, was applied to the column to inhibit proteolytic activity and to remove proteases, DNA and other contaminants, whereas the factor VII still was bound to the column.
- Factor VII was eluted, according to the invention, using the equilibration buffer in which 0.5M arginine was added and adjusted to pH 7.0, to achieve a stable non-aggregated factor VII fraction in a low volume.

Multimodal (or mixed mode) chromatography is a tool for purifying proteins. Described in, for example, Manufacturer data sheet GE Health Care (11-0035-45AA) Capto Adhere, Manufacturer data sheet GE Health Care (28-9078-88AA) Capto MMC and patent application WO-A-2009/024620 "A process for the isolation and purification of a target protein, free of prion proteins".

The aforementioned disadvantage of multimodal chromatography is avoided by mild elution conditions in a pH range about neutral which retains the activity of the vitamin K dependent protein such as FIX molecule and facilitates the use of multi modal chromatography in combination with the stabilisation effects of the increased salt concentration, described in for example in EP-A-1 707 634.

According to one embodiment of the invention, the multi modal chromatography may be performed in a chromatographic column. This may be regarded as a first capture step. The process of the invention can also be performed in a batch mode. The present invention also facilitates a process of purification without addition of human or animal derived stabilizing additives and the use of a whole process which is absent thereof (monoclonal antibody based immuno affinity resins). The use of the multimodal resin, in particular as capture step, facilitates also a higher binding capacity in comparison with conventional ion exchangers, which results in a more concentrated product eluate from the step, which is of advantage for the product stability.

The process of the invention is typically related with the purification of recombinant factor VII.

In another embodiment the process of the invention, the multimodal chromatography step is combined with a factor VII affinity chromatography step wherein the affinity is provided by a protein ligand such as an antibody fragment, which is expressed in yeast.

The invention is further described by the following non-limiting examples.

### Examples

### DESCRIPTION OF ANALYTICAL METHODS

### Comparative - Biological activity analysis - Factor IX (the one-stage clotting assay)

The biological activity of factor IX was measured with a one-stage clotting assay and the unit of factor IX was expressed in International Units (IU) as defined by the current WHO factor IX concentrate standard.

The one-stage clotting assay is the method prescribed in the European Pharmacopoeia. The principle of the assay is based on the ability of a factor IX containing sample to correct the coagulation time of a factor IX deficient plasma in the presence of phospholipids, contact activator and calcium ions. The time of appearance of a fibrin clot is measured in one step. The factor IX activity is inversely proportional to the coagulation time. The method was performed on Siemens BCS XP instrument.

### SDS Page (Molecular weight distribution)

SDS polyacrylamide gel electrophoresis (SDS-PAGE) involves the separation of proteins based on their size. This method describes the SDS-PAGE of proteins, which is run under reduced conditions. By heating the sample under denaturing and reducing conditions, proteins become unfolded and coated with anionic detergent sodium dodecyl sulphate (SDS), acquiring a high net negative charge that is proportional to the length of the polypeptide chain. When loaded onto a polyacrylamide gel matrix and placed in an electric field, the negatively charged protein molecules migrate towards the positively charged electrode and are separated by a molecular sieving effect, i.e. by their molecular weight. Polyacrylamide gels restrain larger molecules from migrating as fast as smaller molecules. Because the charge-to-mass ratio is nearly the same among SDS-denatured polypeptides, the final separation of proteins is dependent almost entirely on the differences in relative molecular mass of polypeptides. In a gel of uniform density, the relative migration distance of a protein (R_{f}) is negatively proportional to the log of its mass. If proteins of known mass are run simultaneously with the unknowns, the relationship between R_{f} and mass can be plotted, and the masses of unknown proteins estimated. The protein bands separated by electrophoresis are visualized by silver staining. Evaluation is done visually by judging the appearances of the standards, reference (control sample) and analysed samples.

### Comparative - Plasma derived Factor IX

The material used in these experiments origins from the commercially available product Nanotiv®, which is a high purity SD treated and nano-filtered Factor IX concentrate.

### Comparative - Recombinant Factor IX

Production of FIX containing cell suspension.

### Cells

The cell line used is a derivative of human embryonic kidney cell 293 (HEK 293), which was adapted to serum-free growth. This host, HEK 293F, was stably transfected with an expression cassette carrying the cDNA coding sequence for human FIX and human furin (PACE). The same strong promoter was used for both cassettes. The general process is also described in EP-A-1 739 179 (Schröder et al.).

### Cultivation method

The cells were cultivated in serum-free medium in general equipment and according to general methods well known in the art, for example shaken or stirred cultures in t-flasks, shaker flasks and bioreactors (disposable systems and conventional stirred tanks) run as batch, fed-batch, perfusion or continuous chemostat cultures (Freshney, R I (2000), Culture of animal cells: a manual of basic technique, 4th ed, Wiley- Liss; Spier, R E ed (2000), Encyclopedia of cell technology, Wiley, New York; Enfors, S-O and Häggström, L (2000), Bioprocess technology: fundamentals and applications, Högskoletryckeriet, Royal Institute of Technology, Stockholm; Vinci, V A and Parekh, S R (2003), Handbook of industrial cell culture: mammalian, microbial, and plant cells, Humana Press, USA). Typically, perfusion of medium was used to increase cell numbers and product titers beyond standard batch culture levels. The product yield and the amount of host cell proteins differ depending on the cultivation mode:
- the product titre will typically increase with cell numbers
- the total protein content and DNA content will typical increase with cell numbers
- the total protein content and DNA content can also increase with culture longevity

- batch cultures accumulate protein and DNA; nothing is externally added, nothing is removed
- perfusion processes rinse cell cultures from metabolites, protein, DNA and other impurities; filters or cell centrifuges were typically used for cell retention.

The recombinant product is released from the cells and the cell suspension or the cell suspension supernatant is the harvest. The properties of the harvest (product titres and impurities as mentioned above) differ depending on the cultivation mode used.

The cell suspension has been used in some of the below described FIX examples.

Purification of FIX using Capto MMC resin as capture step (comparative).

### Comparative Example 1

### Start material

Plasma derived FIX (pdFIX, Nanotiv®) was used. Freeze dried Nanotiv was dissolved and diluted in an equilibration buffer prior to be loaded on a Capto MMC column.

### Chromatographic resin and column

Capto MMC, a mixed mode resin from GE Healthcare (cat no. 17-5317), was use as capture step for the FIX molecule. Capto MMC is a weak cationic resin with hydrophobic and thiophilic interactions and hydrogen bonding. A Tricorn 5/150 column was packed with Capto MMC resin to a bed height of 15.7cm. The column volume (CV) was 3.1ml.

### Buffers

Equilibration buffer: 20mM sodium citrate, 0.1M NaCl, 0.02% Polysorbate 80, pH 7.0
Low salt wash buffer: 20mM sodium citrate, 0.2M NaCl, 0.02% Polysorbate 80, pH 6.5
High salt wash buffer: 20mM sodium citrate, 0.7M NaCl, 0.02% Polysorbate 80, pH 6.5
Eluting buffer: 20mM sodium citrate, 0.2M NaCl, 0.5M arginine mono hydrochloride, 0.02% Polysorbate 80, pH 6.5

### Experimental setup

The column was equilibrated with equilibration buffer followed by loading the start material at a flow rate of 1ml/min. pdFIX bound to the Capto MMC resin during these buffer conditions. As seen in Table 1 no pdFIX was found in the flow through. The resin was thereafter subjected to different washing conditions as described in Table 1 and no pdFIX was detected in any of the tested wash buffers. By adding 0.5 M arginine to the buffer, FIX was eluted from the resin and a yield of 85% was obtained.

**Table 1.**

| Sample | Volume | FIX:C | Total FIX:C | Yield |
|---|---|---|---|---|
| | ml | IU/ml | IU | % |
| Start material | 10 | 25.4 | 254 | 100 |
| Flow through and equil. wash | 40 | 0 | 0 | 0 |
| Low salt wash | 3 | 0 | 0 | 0 |
| High salt wash | 3 | 0 | 0 | 0 |
| Eluate | 6 | 36 | 216 | 85 |

### Conclusion

Plasma derived FIX (pdFIX, Nanotiv) binds to Capto MMC at pH 7 and can be washed with at least 0.7M NaCl buffer without being eluted from the resin.

By addition of 0.5M arginine mono hydrochloride to the buffer, pdFIX is eluted from the column.

### Comparative Example 2

### Start material

Recombinant human FIX (rhFIX) produced in HEK 293 cells. The cells were removed and the cell free supernatant was the start material loaded onto the Capto MMC column.

### Chromatographic resin and column

Capto MMC, a mixed mode resin from GE Healthcare (cat no. 17-5317), was used as capture step for the FIX molecule. Capto MMC is a weak cationic resin with hydrophobic and thiophilic interactions and hydrogen bonding. A XK 26 column was packed with Capto MMC resin to a bed height of 15cm. The column volume (CV) was 80ml.

### Buffers

Equilibration buffer: 20mM sodium citrate, 0.1M NaCl, 0.02% Polysorbate 80, pH 7.0
High salt wash buffer: 20mM sodium citrate, 0.7M NaCl, 0.02% Polysorbate 80, pH 6.5
Eluting buffer: 20mM sodium citrate, 0.2M NaCl, 0.5M arginine mono hydrochloride, 0.02% Polysorbate 80, pH 6.5

### Experimental setup

The column was equilibrated with equilibration buffer followed by loading the start material at a flow rate of 26ml/min. rhFIX bound to the Capto MMC resin during theses buffer conditions. As seen in Table 2 no rhFIX was found in the flow through. The high salt wash did not elute any rhFIX from the column. By adding 0.5M arginine to the buffer rhFIX was eluted from the resin and a yield of 92% was obtained.

**Table 2.**

| Sample | Volume | FIX:C | Total FIX:C | Yield |
|---|---|---|---|---|
| | ml | IU/ml | IU | % |
| Start material | 1600 | 1.27 | 2032 | 100 |
| Flow through and equil. wash | 2400 | 0 | 0 | 0 |
| High salt wash | 400 | 0 | 0 | 0 |
| Eluate | 60 | 31.1 | 1866 | 92 |

### Conclusion

Recombinant FIX (rhFIX) binds to Capto MMC at pH 7 and can be washed with at least 0.7M NaCl buffer without being eluted from the resin.

By addition of 0.5M arginine mono hydrochloride to the buffer, rhFIX is eluted from the column.

The buffers used contained 0.02% Polysorbate 80 (a non-ionic detergent) which did not result in any negative effects. Possibly, it was of an advantage to use Polysorbate 80 in the buffers; compare with the results obtained in the experiment below (Example 3) where no Polysorbate 80 was added to the buffers used.

### Comparative Example 3

### Start material

Recombinant human FIX (rhFIX) was produced in HEK 293 cells. The cells were removed and the cell free supernatant was the start material loaded onto the Capto MMC column.

### Chromatographic resin and column

Capto MMC, a mixed mode resin from GE Healthcare (cat no. 17-5317), was used as capture step for the FIX molecule. Capto MMC is a weak cationic resin with hydrophobic and thiophilic interactions and hydrogen bonding. A XK 26 column was packed with Capto MMC resin to a bed height of 15cm. The column volume (CV) was 80ml.

### Buffers

Equilibration buffer: 20mM sodium citrate, 0.1M NaCl, pH 7.0
High salt wash buffer: 20mM sodium citrate, 0.7M NaCl, pH 6.5
Eluting buffer: 20mM sodium citrate, 0.2M NaCl, 0.8M arginine mono hydrochloride, pH 6.5

### Experiment setup and results

The column was equilibrated with equilibration buffer followed by loading the start material at a flow rate of 26ml/min. rhFIX bound to the Capto MMC resin during theses buffer conditions. As seen in Table 3 a low amount of the rhFIX loaded onto the column was found in the flow through. The resin was thereafter washed with a buffer with a fairly high NaCl concentration, 0.7M. As described in Table 3 less than 1% rhFIX was detected in this wash. By adding 0.8 M arginine to the buffer rhFIX was eluted from the resin and a yield of 84% was obtained. The buffer used in this experiment did not contain any Polysorbate 80 compared to Experiment 2 and Experiment 5.

**Table 3.**

| Sample | Volume | FIX:C | Total FIX:C | Yield |
|---|---|---|---|---|
| | ml | IU/ml | IU | % |
| Start material | 750 | 1.27 | 2032 | 100 |
| Flow through and equil. wash | 1550 | 0 | 0 | 4 |
| High salt wash | 400 | 0 | 0 | 0 |
| Eluate | 60 | 31.1 | 1866 | 84 |

### Conclusion

Recombinant human FIX (rhFIX) binds to Capto MMC at pH 7 and can be washed with at least 0.7M NaCl buffer without being eluted from the resin.

By addition of 0.8M arginine mono hydrochloride to the buffer, rhFIX is eluted from the column.

No detergent was added to the buffers, which can indicate of a somewhat lower recovery of rhFIX in the elution fraction.

### Comparative Example 4 (Example 4A and Example 4B)

### Start material

Plasma derived FIX (pdFIX, Nanotiv®) was used. Freeze dried Nanotiv was dissolved and diluted in an equilibration buffer prior to be loaded on a Capto MMC column.

### Chromatographic resin and column

Capto MMC, a mixed mode resin from GE Healthcare (cat no. 17-5317), was used as capture step for the FIX molecule. Capto MMC is a weak cationic resin with hydrophobic and thiophilic interactions and hydrogen bonding. A Tricorn 5/150 column was packed with Capto MMC resin to a bed height of 15cm. The column volume (CV) was 3ml.

### Buffers

For experiment Example 4A:
Equilibration buffer: 20mM sodium citrate, 0.1M NaCl, pH 7.0
Eluting buffer: 20mM sodium citrate, 0.1M NaCl, 0.5M arginine mono hydrochloride, pH 7.0

For experiment Example 4B:
Equilibration buffer: 20mM sodium citrate, 0.1M NaCl, 0.02% Polysorbate 80, pH 7.0
Eluting buffer: 20mM sodium citrate, 0.1M NaCl, 0.5M arginine mono hydrochloride, 0.02% Polysorbate 80, pH 7.0

### Experimental setup

A comparison of purifying pdFIX on a Capto MMC column with buffers with or without Polysorbate 80 and a pH set at 7.0. The column was equilibrated with equilibration buffer followed by loading the start material at a flow rate of 1ml/min. pdFIX bound to the Capto MMC resin during theses buffer conditions. As seen in Table 4 no pdFIX was found in the flow through and equilibration wash fraction.

**Table 4.**

| Sample | Volume | FIX:C | Total | Yield |
|---|---|---|---|---|
| | ml | IU/ml | FIX:C IU | % |
| Example 4A (without Polysorbate 80) | | | | |
| Start material | 18 | 22.3 | 401 | 100 |
| Flow through and equil. wash | 15 | 0 | 0 | 0 |
| Eluate | 9 | 42.25 | 380 | 95 |
| Example 4B (with Polysorbate 80) | | | | |
| Start material | 18.45 | 25.9 | 478 | 100 |
| Flow through and equil. wash | 42 | 0 | 0 | 0 |
| Eluate | 9 | 52.85 | 476 | 99.5 |

### Conclusion

A 5% increase of plasma derived FIX was eluted when Polysorbate 80 was included in the buffers compared to when no Polysorbate 80 was in the buffers. The difference was low, but the results point towards a benefit of using Polysorbate 80 in the buffers. The yield of pdFIX was high in both experiments. This also shows that pH 7.0 in the buffers used resulted in good data regarding both binding and in the eluting pdFIX from the Capto MMC resin.

### Comparative Example 5 (Example 5A and Example 5B)

### Start material

Plasma derived FIX (Nanotiv) was used. Freeze dried Nanotiv was dissolved and diluted in an equilibration buffer prior to be loaded on a Capto MMC column.

### Chromatographic resin and column

Capto MMC, a mixed mode resin from GE Healthcare (cat no. 17-5317), was use as capture step for the FIX molecule. Capto MMC is a weak cationic resin with hydrophobic and thiophilic interactions and hydrogen bonding. A Tricorn 5/150 column was packed with Capto MMC resin to a bed height of 15cm. The column volume (CV) was 3ml.

### Buffers

For experiment Example 5A:
Equilibration buffer: 20mM HEPES, 0.1M NaCl, pH 8.0
Eluting buffer: 20mM HEPES, 0.1M NaCl, 0.5M arginine mono hydrochloride, pH 8.0

For experiment Example 5B:
Equilibration buffer: 20mM sodium citrate, 0.1M NaCl, pH 6.0
Eluting buffer: 20mM sodium citrate, 0.1M NaCl, 0.5M arginine mono hydrochloride, pH 6.0

### Experimental setup

A comparison of purifying pdFIX on a Capto MMC column with buffers with two different pH, 8.0 and 6.0, used both in the binding and eluting buffers. The column was equilibrated with equilibration buffer followed by loading the start material at a flow rate of 1ml/min. pdFIX bound to the Capto MMC resin during theses buffer conditions. As seen in Table 5 no pdFIX was found in the flow through. The pdFIX yield was equally high in the eluting fraction for both pH tested.

**Table 5.**

| Sample | Volume | FIX:C | Total FIX:C | Yield |
|---|---|---|---|---|
| | ml | IU/ml | IU | % |
| Example 5A (pH 8.0) | | | | |
| Start material | 18.83 | 22.9 | 431 | 100 |
| Flow through and equil. wash | 42 | 0 | 0 | 0 |
| Eluate | 9 | 38.8 | 349 | 81 |
| Example 5B (pH 6.0) | | | | |
| Start material | 17.87 | 14 | 250 | 100 |
| Flow through and equil. wash | 42 | 0 | 0 | 0 |
| Eluate | 15 | 13.6 | 204 | 81 |

### Conclusion

These experiments show that plasma derived FIX can bind to the Capto MMC resin in buffers with pH of 8.0 and 6.0 and that both these pH also can be used in the eluting buffer.

### Comparative Example 6 (Example 6A-6K)

### Aim

The aim of these experiments was to study the capture and elution of recombinant human FIX (rhFIX) on a Capto MMC resin at three different pH: 6.0, 7.0 and 8.0. The benefit of 0.02% Polysorbate 80 in these buffers used regarding binding and elution of rhFIX was also investigated.

### Start material

Recombinant human FIX produced in HEK 293 cells. The cells were removed and the cell free supernatant was the start material loaded onto the Capto MMC column.

### Chromatographic resin and column

Capto MMC, a mixed mode resin from GE Healthcare (cat no. 17-5317), was used as capture step for the FIX molecule. Capto MMC is a weak cationic resin with hydrophobic and thiophilic interactions and hydrogen bonding. A XK 16 column was packed with Capto MMC resin to a bed height of 13.5 cm. The column volume (CV) was 27 ml.

### Buffers

**Comparative Example 6A**

| | |
|---|---|
| Equilibration buffer | 0.1M NaCl, 20mM sodium citrate, pH 7.0 |
| Wash buffer | 0.1M NaCl, 20mM sodium citrate, pH 7.0 |
| Eluting Buffer | 0.1M NaCl, 20mM sodium citrate, 0,5M arginine mono hydrochloride, pH 7.0. |
| CIP | 1% Triton X-100/0.5M NaCl, 1M NaOH, WFI, 20% ethanol |
| Storage buffer | 20% ethanol |

| | |
|---|---|
| WFI: Water for injection | |

**Comparative Example 6B**

| | |
|---|---|
| Equilibration buffer | 0.1M NaCl, 20mM sodium citrate, pH 6.0 |
| Wash buffer | 0.1M NaCl, 20mM sodium citrate, pH 6.0 |
| Eluting Buffer | 0.1M NaCl, 20mM sodium citrate, 0,5M arginine mono hydrochloride, pH 6.0. |
| CIP | 1% Triton X-100/0.5M NaCl, 1M NaOH, WFI, 20% ethanol |
| Storage buffer | 20% ethanol |

**Comparative Example 6C**

| | |
|---|---|
| Equilibration buffer | 0.1M NaCl, 20mM HEPES, pH 8.0 |
| Wash buffer | 0.1M NaCl, 20mM HEPES, pH 8.0 |
| Eluting Buffer | 0.1M NaCl, 20mM HEPES, 0,5M arginine mono hydrochloride, pH 8.0. |
| CIP | 1% Triton X-100/0.5M Na, 1M NaOH, WFI, 20% ethanol |
| Storage buffer | 20% ethanol |

**Comparative Example 6D**

| | |
|---|---|
| Equilibration buffer | 0.1M NaCl, 20mM sodium citrate, 0.02% Polysorbate 80, pH 7.0 |
| Wash buffer | 0.1M NaCl, 20mM sodium citrate, 0.02% Polysorbate 80, pH 7.0 |
| Eluting Buffer | 0.1M NaCl, 20mM sodium citrate, 0,5M arginine mono hydrochloride, 0.02% Polysorbate 80, pH 7.0. |
| CIP | 1% Triton X-100/0.5M NaCl, 1M NaOH, WFI, 20% ethanol |
| Storage buffer | 20% ethanol |

**Comparative Example 6E**

| | |
|---|---|
| Equilibration buffer | 0.1M NaCl, 20mM sodium citrate, 0.02% Polysorbate 80, pH 6.0 |
| Wash buffer | 0.1M NaCl, 20mM sodium citrate, 0.02% Polysorbate 80, pH 6.0 |
| Eluting Buffer | 0.1M NaCl, 20mM sodium citrate, 0,5M arginine mono hydrochloride, 0.02% Polysorbate 80, pH 6.0. |
| CIP | 1% Triton X-100/0.5M NaCl, 1M NaOH, WFI, 20% ethanol |
| Storage buffer | 20% ethanol |

**Comparative Example 6F**

| | |
|---|---|
| Equilibration buffer | 0.1M NaCl, 20mM HEPES, pH 8.0 |
| Wash buffer | 0.1M NaCl, 20mM HEPES, pH 8.0 |
| Eluting Buffer | 0.1M NaCl, 20mM HEPES, 0,5M arginine mono hydrochloride, pH 8.0. |
| CIP | 1% Triton X-100/0.5M NaCl, 1M NaOH, WFI, 20% ethanol |
| Storage buffer | 20% ethanol |

### Experimental setup and results

The column was equilibrated with equilibration buffer followed by loading the start material at a flow rate of 9ml/min. The column was then washed with equilibration buffer followed by eluting the bound rhFIX from the Capto MMC column. The results are presented in Table 6.

**Table 6.**

| Exp. No. Example | pH | 0.02% P80 | FIX in the Flow through % | FIX in the Eluate % |
|---|---|---|---|---|
| Example 6A (comparative) | 7.0 | No | 0 | 89 |
| Example 6D (comparative) | 7.0 | Yes | 0 | 94 |
| Example 6B (comparative) | 6.0 | No | 0 | 77 |
| Example 6E (comparative) | 6.0 | Yes | 0 | 86 |
| Example 6C (comparative) | 8.0 | No | 0 | 80 |
| Example 6F (comparative) | 8.0 | Yes | 0 | 78 |

The data presented in Table 6 shows that rhFIX binds to Capto MMC at pH 6, pH 7 and pH 8. No rhFIX was found in the flow though. The bound rhFIX can also be eluted from the Capto MMC resin at these pH with a recovery of >75%. The best recovery was obtained with n eluting buffer with pH 7. The addition of Polysorbate 80 in the equilibration and eluting buffer resulted in an increase of at least 5% when pH 7 or 6 was used. No benefit was obtained for Polysorbate 80 when buffers with pH 8 was used.

These data and the data obtained in experiment 5 indicate for a positive effect of Polysorbate 80 for the recovery of rhFIX and pdFIX on a Capto MMC resin.

### Conclusion

Recombinant human FIX can bind to Capto MMC at a pH range of at least 6-8. The rhFIX molecules are eluted from the Capto MMC resin by addition of 0.5M arginine mono hydrochloride to the buffers independent of which of the three tested pH that was used.

But the results obtained shows that pH 7 results in a larger rhFIX yield.

The addition of the detergent Polysorbate 80 indicates for an increase of the rhFIX recovery when pH 7 or 6 was used.

### Comparative Example 7

### Aim

The aim of this work was to purify recombinant human FIX from cultivation media to a pure product. This included three steps; a capture step, an affinity step and a concentrating and buffer-exchanging step. A Capto MMC resin was used as a capture step and a non-animal derived FIX affinity ligand step was used as the major purification step. As the final step an ultra-filtration system with a 10kDa cut off was used, first to concentrate the molecule and then to exchange the buffer to a physiological buffer environment.

### Start material for the capture step

Recombinant human FIX produced in HEK 293 cells. The cells were removed and the cell free supernatant was the start material loaded onto the Capto MMC column.

### Capture step (Comparative Example 7A-C)

Capto MMC, a mixed mode resin from GE Healthcare (cat no. 17-5317), was used as capture step for the FIX molecule. Capto MMC is a weak cationic resin with hydrophobic and thiophilic interactions and hydrogen bonding. A XK 50 column was packed with Capto MMC resin to a bed height of 16.5 cm. The column volume (CV) was 324 ml.

| | |
|---|---|
| Equilibration buffer | 0.1M NaCl, 20mM sodium citrate, pH 7.0 |
| Wash buffer | 0.1M NaCl, 20mM sodium citrate, pH 7.0 |
| Eluting Buffer | 0.1M NaCl, 20mM sodium citrate, 0,5M arginine mono hydrochloride, pH 7.0. |
| CIP | 1% Triton X-100/0.5M NaCl, 1M NaOH, WFI, 20% ethanol |
| Storage buffer | 20% ethanol |

### Experimental setup and results

The column was equilibrated with equilibration buffer followed by loading the start material at a flow rate of 75ml/min. The column was then washed with equilibration buffer followed by eluting the bound rhFIX from the Capto MMC column. The results are presented in Table 7. The purification of rhFIX was performed in three separate runs, using the same experimental set up and the same type of buffers. These capture purifications were performed at pH 7.0.

**Table 7.**

| Experiment | FIX in the Flow through % | FIX in the Eluate % |
|---|---|---|
| Example 7A (comparative) | 0 | 84.5 |
| Example 7B (comparative) | 2 | 93.5 |
| Example 7C (comparative) | 0 | 100 |

The data presented in Table 7 shows that rhFIX binds to Capto MMC at pH 7. No or very little rhFIX was found in the flow though. The bound rhFIX could also be eluted from the Capto MMC resin at pH 7.0 by adding arginine-HCI to a final concentration of 0.5M. The average recovery of these three captures of rhFIX was 92%.

### FIX Affinity step (Comparative Example 7D-E)

As an affinity step, a non-animal derived FIX affinity ligand (Fab fragment) produced in yeast was used (developed in co-operation with BAC BV, the Bio Affinity Company). This ligand was coupled to a Capto MP resin (GE Healthcare) according to standard coupling techniques and is referred to as "the FIX affinity resin".

A XK 26 column was packed with the FIX affinity resin to a bed height of 7.5 cm. The column volume (CV) was 40 ml.

| | |
|---|---|
| Equilibration buffer | 0.15M NaCl, 10mM sodium citrate, pH 7.0 |
| Wash buffer | 0.15M NaCl, 10mM sodium citrate, pH 7.0 |
| Eluting Buffer | 2M MgCl2, 20mM Tris base, pH 7.4 |
| CIP | 0.1M HAc, 2M NaCl and 20% ethanol |
| Storage buffer | 20% ethanol |

### Experimental setup and results

The eluates from the three capture steps were pooled and used as start material on the affinity column. The column was equilibrated with equilibration buffer followed by loading the start material at a flow rate of 10ml/min. The column was then washed with equilibration buffer followed by eluting the bound rhFIX from the affinity column. The results are presented in Table 8. The purification of rhFIX was performed in two separate runs, using the same experimental set up and the same type of buffers.

**Table 8**

| Experiment | FIX, % Flow through | FIX, % Eluate |
|---|---|---|
| Example 7D (comparative) | 50 | 47 |
| Example 7E (comparative) | 15 | 80 |

The data presented in Table 8 shows that rhFIX binds to FIX affinity resin at pH 7. The flow through obtained was an effect of the binding capacity of the amount of resin used. Approximately 100% of the rhFIX used in both experiments are detectable. The bound rhFIX could be eluted from FIX affinity resin by using a buffer containing 2M MgCl2.

### Concentrating and buffer exchange step (Comparative Example 7F)

By using a UF system, the rhFIX in the eluate from the affinity column was concentrated and then the buffer was exchanged by a dia-filtration using the same UF filter.

### UF filter

A Pellicon 3 filter with a 10kDa cut off mounted in a Pellicon 2 system was used.

| | |
|---|---|
| Diafiltration buffer | 0.15M NaCl, 20mM sodium citrate, pH 7.0 Conductivity 18.4mS/cm at 25°C. |

### Experimental setup and results

By pumping the sample in a tangential flow and allowing a small part of the volume continuously passing through the Pellicon 3 filter, the volume was decreased. Because of the pore size used in the filter, the rhFIX molecule was contained in the re-circulating fraction. The Molecular weight of rhFIX is approximately 55kDa and is retained when a filter with a pore size of 10kDa is used.

A volume of 310ml affinity eluate was concentrated to approximately 60ml. This volume was the dia-filtrated by continuously adding dia-filtration buffer at the same speed as is filtered through the Pellicon 3 filter. The buffer in this 60ml concentrate was exchanged approximately 18 times. The conductivity of this concentrate was changed from 123mS/cm to 18.6mS/cm at 25 °C.

The rhFIX recovery over this concentration and buffer exchange step was 86%. A wash step of the UF filter with the dialysis buffer was performed to recover as much rhFIX as possible.

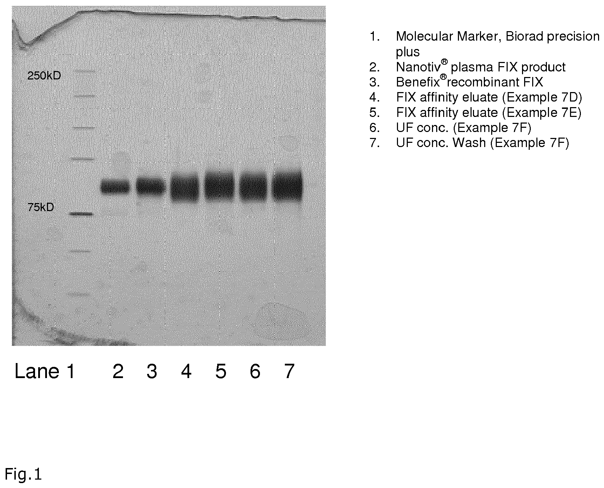

Fig. 1 shows the SDS Page purity pattern after the FIX affinity resin purification. Lane 1 is a commercially available molecular marker, Lane 2 is a commercially available high purity plasma derived FIX product, Lane 3 is a commercially available high purity recombinant FIX product, Lane 4 and 5 are samples after FIX affinity purification of the invention as described in Example 7D and 7E, Lane 6 and 7 are samples after FIX affinity purification and ultrafiltration concentration/ desalting as described in Example 7F. As can be seen from Lane 4-7 the FIX purity of the product produced according to the invention do not show impurities of lower or higher molecular weight as compared with Lane 2 or 3.

## Claims

1. A process of manufacturing a prion-free Vitamin K dependent Protein in a purification sequence employing chromatography **characterized in that**
- at least one chromatography step is performed using a multimodal resin;
- providing a fraction containing Vitamin K dependent Protein in an aqueous solution;
- contacting the fraction containing the Vitamin K dependent Protein with a multimodal resin at a pH between 6-9;
- optionally washing the multimodal resin having the Vitamin K dependent Protein adsorbed with an aqueous washing buffer to wash away contaminants and retain the Vitamin K dependent Protein, before the Vitamin K dependent Protein is eluted;
- the Vitamin K dependent Protein is eluting from the multimodal resin at a pH between 6 to 9 in a buffer comprising arginine; and
- optionally collecting Vitamin K dependent Protein containing fractions in purified or enriched form,
wherein the Vitamin K dependent Protein is factor VII.

2. The process of claim 1, wherein the multimodal resin comprises moieties bound to a matrix and the moieties are able to interact with the Vitamin K dependent Protein in an aqueous environment by ionic interactions and other types of interactions such as hydrogen bonding, hydrophobic and thiophilic interactions.

3. The process of claim 1 or claim 2, **characterised in that** the aqueous solution comprises the Vitamin K dependent Protein in a salt solution corresponding to a conductivity of from about 5 to about 200 mS/cm at 25°C and/or the elution buffer corresponding to a conductivity of from about 5 to about 200 mS/cm at 25 °C.

4. The process of any one of claims 1 to 3, **characterised in that** arginine is present in concentrations of 0.1 - 0.3 M and 0.7 - 1 M, in particularly 0.3 - 0.7M.

5. The process of claim 4, **characterised in that** the Vitamin K dependent Protein is eluted with a buffer having a pH of about 6 to about 9, in particular pH about 6 to about 8 or pH 7.

6. The process of any one of claims 1 to 5, **characterised in that** the elution buffer is additionally comprising agents selected from the group consisting of at least one hydroxyl group containing organic compounds such as an alcohol, at least one amino group containing organic compound such as an amino acid, at least one compound for regulating the ionic strength of the buffer such as an inorganic salts selected from the group consisting of of sodium chloride, sodium phosphate, sodium sulphate, potassium chloride, potassium phosphate, potassium sulphate, magnesium chloride, calcium chloride, barium chloride, barium sulphate, ammonium sulphate at least one non-ionic detergent, at least one buffering substance to regulate the pH from about 6 to about 9 in particular to about a neutral value, or combinations thereof.

7. The process of claim 6, **characterised in that** the alcohol is selected from the group consisting of methanol, propanol, ethylene glycol and propylene glycol; the inorganic salt is selected from the group consisting of KCI and NaCl; the non-ionic detergent is selected from the group consisting of Tween 20, Tween 80 and Pluronic F68; the buffering substance is selected from the group consisting of sodium citrate, histidine, HEPES, MES, Tris base and sodium acetate at a pH between 6-9, sodium citrate for adjustment of pH 6-7.5 and Tris base for adjustment of pH 7.5 - 9.

8. The process of any one of claims 1 to 7, wherein the Vitamin K dependent Protein is eluted with a combination of NaCI/Ethylene glycol or combination of arginine/NaCl, or combination of arginine/Ethylene glycol.

9. The process of any one of claims 1 to 8, **characterised in that** the "multimodal" chromatography resin contains at least one of the following moieties:
a. a positively charged N-Benzyl-N-methyl ethanolamine ligand,
b. a negatively charged 2- (benzoylamino) butanoic acid ligand,
c. a phenylpropyl ligand,
d. a N-hexyl ligand,
e. a 4-Mercapto-Ethyl-Pyridine ligand,
f. a 3-((3-methyl-5- ((tetrahydrofuran-2-ylmethyl)-amino)-phenyl)-amino)-benzoic acid ligand or combinations thereof.

10. The process of any one of claims 1 to 9, **characterised in that** the "multimodal" chromatography resin is selected from the following commercially available resins HEP Hypercel™; PPA Hypercel™; Capto Adhere™; Capto MMC™; MEP Hypercel™.

11. The process of any one of claims 1 to 10, **characterised in that** the multimodal chromatography step is combined with a the Vitamin K dependent Protein affinity chromatography step wherein the affinity is provided by a ligand which is based on a protein expressed in yeast and the purity of the resulting eluate is more than 90%.

12. The process of any one of claims 1 to 11, **characterised in that** the purification sequence further comprises pathogen removal/inactivation steps comprising a chemically based inactivation step, ultra filtration, chromatography steps or combinations thereof which steps are based on different physiological properties directed to the pathogen to be removed.

13. The process of any one of claims 1 to 12, **characterised in that** the purification sequence further comprises the following steps:
i. a cation multimodal resin such as Capto MMC;
ii. a chemically based inactivation step for lipid enveloped viruses in particular the solvent/detergent-inactivation employing tri-n- butyl phosphate and Triton X-100;
iii. an affinity resin based on a protein ligand such as a, ligand consisting of an Vitamin K dependent Protein antibody fragment expressed in yeast;
iv. a pathogen filtration removal step with a mean pore size of about 20 nm such as Planova 20N;
v. an anion exchanger resin such as Q Sepharose FF or Capto Q;
vi. a ultra filtration step such as Pellicon 3 with a cut off 10kDa.

14. The process of claim 13, **characterised in that** the multimodal chromatography step is combined with an affinity chromatography step wherein the affinity is provided by a ligand which is based on a protein expressed in yeast and that the purity of resulting product from the affinity chromatography resin is more than 95%.

15. The process according to claim 14, **characterised in that** additional chromatography step(s) is/are performed, selected from size exclusion, anion exchange, cation exchange, hydrophobic interaction and immobilized metal affinity chromatography, **characterised in that** the purity of the final product is more than 99%.

## Patentansprüche

1. Ein Verfahren zur Herstellung eines prionenfreien, Vitamin-K-abhängigen Proteins in einer Aufreinigungssequenz mittels Chromatographie, **dadurch gekennzeichnet, dass**
- mindestens ein Chromatographieschritt unter Verwendung eines multimodalen Harzes durchgeführt wird;
- Bereitstellen einer Fraktion, die das Vitamin-K-abhängige Protein in einer wässrigen Lösung enthält;
- Inkontaktbringen der Fraktion, die das Vitamin-K-abhängige Protein enthält, mit einem multimodalen Harz bei einem pH zwischen 6-9;
- gegebenenfalls Waschen des multimodalen Harzes, das das Vitamin-K-abhängige Protein adsorbiert hat, mit einem wässrigen Waschpuffer, um Verunreinigungen wegzuwaschen und das Vitamin-K-abhängige Protein zu behalten, bevor das Vitamin-K-abhängige Protein eluiert wird;
- das Vitamin-K-abhängige Protein von dem multimodalen Harz bei einem pH-Wert zwischen 6 bis 9 in einem Puffer umfassend Arginin eluiert wird; und
- gegebenenfalls Sammeln von Fraktionen, die das Vitamin-K-abhängige Protein in gereinigter oder angereicherter Form enthalten,
wobei das Vitamin-K-abhängige Protein Faktor VII ist.

2. Das Verfahren gemäß Anspruch 1, wobei das multimodale Harz an eine Matrix gebundene Einheiten umfasst und die Einheiten mit dem Vitamin-K-abhängigen Protein in einer wässrigen Umgebung durch ionische Wechselwirkungen und andere Arten von Wechselwirkungen wie Wasserstoffbrücken, hydrophobe und thiophile Wechselwirkungen interagieren können.

3. Das Verfahren gemäß Anspruch 1 oder Anspruch 2, **dadurch gekennzeichnet, dass** die wässrige Lösung das Vitamin-K-abhängige Protein in einer Salzlösung mit einer entsprechenden Leitfähigkeit von etwa 5 bis etwa 200 mS/cm bei 25°C und/oder dem Elutionspuffer entsprechend einer Leitfähigkeit von etwa 5 bis etwa 200 mS/cm bei 25°C umfasst.

4. Das Verfahren gemäß einem der Ansprüche 1 bis 3, **dadurch gekennzeichnet, dass** Arginin in Konzentrationen von 0,1 - 0,3 M und 0,7 - 1 M vorliegt, insbesondere 0,3 - 0,7 M.

5. Das Verfahren gemäß Anspruch 4, **dadurch gekennzeichnet, dass** das Vitamin-K-abhängige Protein mit einem Puffer eluiert wird, der einen pH von ungefähr 6 bis ungefähr 9 hat, insbesondere einen pH von ungefähr 6 bis ungefähr 8 oder pH 7.

6. Das Verfahren gemäß einem der Ansprüche 1 bis 5, **dadurch gekennzeichnet, dass** der Elutionspuffer zusätzlich Agenzien umfasst ausgewählt aus der Gruppe bestehend aus mindestens einer Hydroxylgruppen-enthaltenden organischen Verbindung wie etwa einem Alkohol, mindestens einer Aminogruppen-enthaltende organische Verbindung wie etwa einer Aminosäure, mindestens einer Verbindung zur Regulierung der lonenstärke des Puffers wie etwa einem anorganischen Salz, ausgewählt aus der Gruppe bestehend aus Natriumchlorid, Natriumphosphat, Natriumsulfat, Kaliumchlorid, Kaliumphosphat, Kaliumsulfat, Magnesiumchlorid, Kalziumchlorid, Bariumchlorid, Bariumsulfat, Ammoniumsulfat, mindestens einem nichtionischen Detergens, mindestens einer Puffersubstanz, um den pH von ungefähr 6 bis ungefähr 9, insbesondere bis ungefähr einem neutralen Wert zu regulieren, oder Kombinationen davon.

7. Das Verfahren gemäß Anspruch 6, **dadurch gekennzeichnet, dass** der Alkohol ausgewählt ist aus der Gruppe bestehend aus Methanol, Propanol, Ethylenglykol und Propylenglykol; das anorganische Salz ausgewählt ist aus der Gruppe bestehend aus KCl und NaCl; das nicht-ionische Detergens ausgewählt ist aus der Gruppe bestehend aus Tween 20, Tween 80 und Pluronic F68; die Puffersubstanz ausgewählt ist aus der Gruppe bestehend aus Natrium-Citrat, Histidin, HEPES, MES, Tris-Base und Natrium-Acetat bei einem pH zwischen 6-9, Natrium-Citrat zur Einstellung von pH 6-7,5 und Tris-Base zur Einstellung von pH 7,5-9.

8. Das Verfahren gemäß einem der Ansprüche 1 bis 7, wobei das Vitamin-K-abhängige Protein mit einer Kombination aus NaCl/Ethylenglykol oder einer Kombination aus Arginin/NaCl oder einer Kombination aus Arginin/ Ethylenglykol eluiert wird.

9. Das Verfahren gemäß einem der Ansprüche 1 bis 8, **dadurch gekennzeichnet, dass** das "mulimodale" Chromatographieharz mindestens eine der folgenden Einheiten enthält:
a. einen positiv geladenen N-Benzyl-N-methyl-ethanolamin-Liganden,
b. einen negativ geladenen 2-(Benzoylamino)-Buttersäure-Liganden,
c. einen Phenylpropyl-Liganden,
d. einen N-Hexyl-Liganden
e. einen 4-Mercapto-ethyl-pyridin-Liganden,
f. einen 3-((3-Methyl-5-((tetrahydrofuran-2-yl-methyl)-amino)-phenyl)-amino)-Benzoesäure-Liganden oder Kombinationen davon.

10. Das Verfahren gemäß einem der Ansprüche 1 bis 9, **dadurch gekennzeichnet, dass** das "multimodale" Chromatographieharz ausgewählt ist aus den folgenden kommerziell erhältlichen Harzen HEP Hypercel™; PPA Hypercel™; Capto Adhere™; Capto MMC™; MEP Hypercel™.

11. Das Verfahren gemäß einem der Ansprüche 1 bis 10, **dadurch gekennzeichnet, dass** der multimodale Chromatographieschritt mit einem Vitamin-K-abhängigen-Protein-Affinitätschromatographieschritt kombiniert wird, wobei die Affinität durch einen Liganden bereitgestellt wird, der auf einem in Hefe exprimierten Protein basiert und die Reinheit des resultierenden Eluats mehr als 90% ist.

12. Das Verfahren gemäß einem der Ansprüche 1 bis 11, **dadurch gekennzeichnet, dass** die Aufreinigungssequenz weiterhin Pathogenentfernungs-/ inaktivierungsschritte umfasst, die einen chemisch-basierten Inaktivierungsschritt, Ultrafiltration, Chromatographieschritte oder Kombinationen davon umfassen, wobei die Schritte auf den verschiedenen physiologischen Eigenschaften des zu entfernenden Pathogens basieren.

13. Das Verfahren gemäß einem der Ansprüche 1 bis 12, **dadurch gekennzeichnet, dass** die Aufreinigungssequenz weiterhin die folgenden Schritte umfasst:
i. ein Kationen-multimodales Harz wie etwa Capto MMC;
ii. einen chemisch-basierten Inaktivierungsschritt für lipidbehüllte Viren, insbesondere die Lösungsmittel/Detergens-Inaktivierung durch Tri-n-butyl-phosphat und Triton X-100;
iii. ein Affinitätsharz, das auf einem Proteinliganden wie etwa einem Liganden bestehend aus einem in Hefe exprimierten Vitamin-K-abhängigen Protein-Antikörperfragment basiert;
iv. einen Pathogenfiltrations-Entfernungsschritt mit einer durchschnittlichen Porengröße von etwa 20 nm wie Planova 20N;
v. ein Anionenaustauscherharz wie Q Sepharose FF oder Capto Q;
vi. einen Ultrafiltrationsschritt wie etwa Pellicon 3 mit einer Ausschlussgrenze von 10 kDa.

14. Das Verfahren gemäß Anspruch 13, **dadurch gekennzeichnet, dass** der multimodale Chromatographieschritt mit einem Affinitätschromatographieschritt kombiniert wird, wobei die Affinität durch einen Liganden bereitgestellt wird, der auf einem in Hefe exprimierten Protein basiert und die Reinheit des resultierenden Produkts von dem Affinitätschromatographieharz mehr als 95% ist.

15. Das Verfahren gemäß Anspruch 14, **dadurch gekennzeichnet, dass** (ein) zusätzliche(r) Chromatographieschritt(e) durchgeführt wird/werden ausgewählt aus Größenausschluss-, Anionenaustausch-, Kationenaustausch-, Hydrophobe-Interaktionen- und Immobilisierte-Metallionen-Affinitätschromatographie, **dadurch gekennzeichnet, dass** die Reinheit des Endprodukts mehr als 99% ist.

## Revendications

1. Procédé de fabrication d'une protéine vitamine K-dépendante exempte de prions dans une séquence de purification employant une chromatographie, **caractérisé en ce que**
- au moins une étape de chromatographie est réalisée en utilisant une résine multimodale ;
- une fraction contenant une protéine vitamine K-dépendante dans une solution aqueuse est mise à disposition ;
- la fraction contenant la protéine vitamine K-dépendante est mise en contact avec une résine multimodale à un pH compris entre 6-9 ;
- la résine multimodale comportant la protéine vitamine K-dépendante adsorbée est facultativement lavée avec un tampon de lavage aqueux pour éliminer les contaminants et retenir la protéine vitamine K-dépendante, avant que la protéine vitamine K-dépendante ne soit éluée ;
- la protéine vitamine K-dépendante est éluée de la résine multimodale à un pH compris entre 6 et 9 dans un tampon comprenant de l'arginine ; et
- les fractions contenant la protéine vitamine K-dépendante sont facultativement collectées sous une forme purifiée ou enrichie,
dans lequel la protéine vitamine K-dépendante est le facteur VII.

2. Procédé selon la revendication 1, dans lequel la résine multimodale comprend des fragments liés à une matrice et les fragments sont capables d'interagir avec la protéine vitamine K-dépendante dans un environnement aqueux par des interactions ioniques et d'autres types d'interactions telles que des liaisons hydrogène, des interactions hydrophobes et thiophiliques.

3. Procédé selon la revendication 1 ou la revendication 2, **caractérisé en ce que** la solution aqueuse comprend la protéine vitamine K-dépendante dans une solution de sel correspondant à une conductivité d'environ 5 à environ 200 mS/cm à 25 °C et/ou le tampon d'élution correspondant à une conductivité d'environ 5 à environ 200 mS/cm à 25 °C.

4. Procédé selon l'une quelconque des revendications 1 à 3, **caractérisé en ce que** l'arginine est présente en des concentrations de 0,1-0,3 M et 0,7-1 M, en particulier 0,3-0,7 M.

5. Procédé selon la revendication 4, **caractérisé en ce que** la protéine vitamine K-dépendante est éluée avec un tampon possédant un pH d'environ 6 à environ 9, en particulier un pH d'environ 6 à environ 8 ou un pH 7.

6. Procédé selon l'une quelconque des revendications 1 à 5, **caractérisé en ce que** le tampon d'élution comprend en outre des agents sélectionnés dans le groupe consistant en des composés organiques contenant au moins un groupe hydroxyle tels qu'un alcool, des composés organiques contenant au moins un groupe amino tels qu'un acide aminé, au moins un composé pour réguler la force ionique du tampon tel qu'un sel inorganique sélectionné dans le groupe consistant en le chlorure de sodium, le phosphate de sodium, le sulfate de sodium, le chlorure de potassium, le phosphate de potassium, le sulfate de potassium, le chlorure de magnésium, le chlorure de calcium, le chlorure de baryum, le sulfate de baryum, le sulfate d'ammonium, au moins un détergent non ionique, au moins une substance tampon afin de réguler le pH entre environ 6 et environ 9, en particulier à environ une valeur neutre, ou des combinaisons de ceux-ci.

7. Procédé selon la revendication 6, **caractérisé en ce que** l'alcool est sélectionné dans le groupe consistant en le méthanol, le propanol, l'éthylène glycol et le propylène glycol ; le sel inorganique est sélectionné dans le groupe consistant en le KCl et le NaCl ; le détergent non ionique est sélectionné dans le groupe consistant en le Tween 20, le Tween 80 et le Pluronic F68 ; la substance tampon est sélectionnée dans le groupe consistant en le citrate de sodium, l'histidine, l'HEPES, le MES, une base Tris et l'acétate de sodium à un pH compris entre 6-9, le citrate de sodium pour l'ajustement du pH 6-7,5 et la base Tris pour l'ajustement du pH 7,5-9.

8. Procédé selon l'une quelconque des revendications 1 à 7, dans lequel la protéine vitamine K-dépendante est éluée avec une combinaison de NaCl/éthylène glycol ou une combinaison d'arginine/NaCl, ou une combinaison d'arginine/éthylène glycol.

9. Procédé selon l'une quelconque des revendications 1 à 8, **caractérisé en ce que** la résine de chromatographie « multimodale » contient au moins un des fragments suivants :
a. un ligand N-benzyl-N-méthyléthanolamine positivement chargé,
b. un ligand acide 2-(benzoylamino)butanoïque négativement chargé,
c. un ligand phénylpropyle,
d. un ligand N-hexyle,
e. un ligand 4-mercapto-éthyl-pyridine,
f. un ligand acide 3-((3-méthyl-5-((tétrahydrofuran-2-ylméthyl)-amino)-phényl)-amino)-benzoïque ou des combinaisons de ceux-ci.

10. Procédé selon l'une quelconque des revendications 1 à 9, **caractérisé en ce que** la résine de chromatographie « multimodale » est sélectionnée parmi les résines disponibles dans le commerce suivantes : HEP Hypercel™ ; PPA Hypercel™ ; Capto Adhere™ ; Capto MMC™ ; MEP Hypercel™.

11. Procédé selon l'une quelconque des revendications 1 à 10, **caractérisé en ce que** l'étape de chromatographie multimodale est combinée avec une étape de chromatographie d'affinité de la protéine vitamine K-dépendante dans laquelle l'affinité est conférée par un ligand qui est basé sur une protéine exprimée dans une levure et la pureté de l'éluat résultant est supérieure à 90 %.

12. Procédé selon l'une quelconque des revendications 1 à 11, **caractérisé en ce que** la séquence de purification comprend en outre des étapes d'élimination/d'inactivation d'agents pathogènes comprenant une étape d'inactivation chimique, une ultrafiltration, des étapes de chromatographie ou des combinaisons de celles-ci, lesquelles étapes sont basées sur différentes propriétés physiologiques dirigées contre l'agent pathogène à éliminer.

13. Procédé selon l'une quelconque des revendications 1 à 12, **caractérisé en ce que** la séquence de purification comprend en outre les étapes suivantes :
i. une résine multimodale cationique telle que Capto MMC ;
ii. une étape d'inactivation chimique pour les virus à enveloppe lipidique, en particulier l'inactivation au solvant/détergent employant du phosphate de tri-n-butyle et du Triton X-100 ;
iii. une résine d'affinité basée sur un ligand protéique tel qu'un ligand consistant en un fragment d'anticorps anti-protéine vitamine K-dépendante exprimé dans une levure ;
iv. une étape d'élimination par filtration d'agents pathogènes avec une taille de pore moyenne d'environ 20 nm telle que Planova 20N ;
v. une résine échangeuse d'anions telle que Q Sepharose FF ou Capto Q ;
vi. une étape d'ultrafiltration telle que Pellicon 3 avec un seuil de coupure de 10 kDa.

14. Procédé selon la revendication 13, **caractérisé en ce que** l'étape de chromatographie multimodale est combinée avec une étape de chromatographie d'affinité dans laquelle l'affinité est conférée par un ligand qui est basé sur une protéine exprimée dans une levure et **en ce que** la pureté du produit résultant de la résine de chromatographie d'affinité est supérieure à 95 %.

15. Procédé selon la revendication 14, **caractérisé en ce que** une/des étape(s) de chromatographie supplémentaire(s) est/sont réalisée(s), sélectionnées parmi une chromatographie d'exclusion stérique, échangeuse d'anions, échangeuse de cations, d'interaction hydrophobe et d'affinité sur ions métalliques immobilisés, **caractérisé en ce que** la pureté du produit final est supérieure à 99 %.
